# EUROPEAN PATENT SPECIFICATION

(11) **EP 2 373 323 B1**
(45) Date of publication and mention of the grant of the patent: **14.08.2013**
(21) Application number: 09759905.4
(22) Date of filing: 27.11.2009
(51) Int. Cl.: A61K 36/185, A61P 31/12

(54) **Aqueous extract from Ribes for treatment of viral infections**
Wässriger Extrakt aus Ribes zur Behandlung von Virusinfektionen
Extrait aqueux de Ribes pour le traitement d'infections virales

(30) Priority: 12.12.2008 EP 08021663
(43) Date of publication of application: 12.10.2011
(73) Proprietor: Pandalis, Georgios, 49219 Glandorf (DE)
(72) Inventor: Pandalis, Georgios, 49219 Glandorf (DE)
(74) Representative: Grünecker, Kinkeldey, Stockmair & Schwanhäusser
(86) International application number: PCT/EP2009/008471
(87) International publication number: WO 2010/066346

(56) References cited:
- US-A1- 2005 266 104
- TABART ET AL: "Optimisation of extraction of phenolics and antioxidants from black currant leaves and buds and of stability during storage" FOOD CHEMISTRY, vol. 105, 2007, pages 1268-1275, XP022158242
- KNOX ET AL: "Activity of anthocyanins from fruit extract of Ribes nigrum L. against influenza A and B viruses" ACTA VIROLOGICA, vol. 45, 2001, pages 209-215, XP009065720
- TABART ET AL: "Antioxidant capacity of black currant varies with organ, season, and cultivar" JOURNAL OF AGRICULTURAL AND FOOD CHEMISTRY, vol. 54, 2006, pages 6271-6276, XP002525885
- KNOX ET AL: "Anti-influenza virus activity of crude extract of Ribes nigrum L." PHYTOTHERAPY RESEARCH, vol. 17, 2003, pages 120-122, XP002525886
- SUZUTANI ET AL: "Anti-herpesvirus activity of an extract of Ribes nigrum" PHYTOTHERAPY RESEARCH, vol. 17, 2003, pages 609-613, XP002525887
- MIDDLETON ET AL: "The effects of plant flavonoids on mammalian cells: Implications for inflammation, heart disease, and cancer" PHARMACOLOGICAL REVIEWS, vol. 52, 2000, XP002526090
- BRINKWORTH ET AL: "Flavones are inhibitors of HIV-1 proteinase" BIOCHEMICAL AND BIOPHYSICAL RESEARCH COMMUNICATIONS, vol. 188, 1992, pages 631-637, XP024770368
- DATABASE WPI Thomson Scientific, London, GB;page 1-2, AN 2002-297465 XP002602899 "Antiviral agent which has antiviral activity against influenza virus, and for use as trochiscus, mouth wash, jam, confectionary and beverages, comprises cyanidin glycoside" & JP 2001 328941 A (TANIUCHI) 27 November 2001 (2001-11-27)
- GHEDIRA ET AL: "Ribes nigrum L." PHYTOTHÉRAPIE, vol. 6, April 2008 (2008-04), pages 125-128, XP019596426
- DATABASE FSTA INTERNATIONAL FOOD INFORMATION SERVICE (IFIS), FRANKFURT AM MAIN, GERMANYE; Abstract number: 77-3-05-a0336, 1976, KOROVKA: "Content of ascorbic acid in edible wild plants in the Komi-Permi region" XP002525933
- DATABASE BIOSIS BIOSCIENCES INFORMATION SERVICE, PHILADELPHIA, PA, US; Abstract number: PREV199192061942, 1991, FOKINA ET AL: "Experimental phytotherapy of tick-borne encephalitis" XP002525934

## Description

The present invention relates to a composition comprising an extract of the genus Ribes for use in the prophylaxis and/or treatment of viral infections.

A great number of human diseases are caused by viruses and include influenza, the common cold, chickenpox and cold sores. Diseases like AIDS, hepatitis, herpes infections, Coxsackie infections, measles, rubella, cytomegaly, mumps, rabies, diarrhea, SARS, Ebola, Yellow fever, West-Nile fever, Hanta fever, Dengue fever, Marburg fever, Lassa fever, smallpox, human papillomaviral infections, infectious mononucleosis, Burkitt lymphoma, polyomyelitis, encephalitits, adenopharyngitis are also virus-borne.

However, the most common viral infections in humans are influenza and common cold diseases. Each year about 10 to 20% of the world population fall ill due to influenza, and common cold diseases are the most frequent infection of humans at all. Usually adults are affected two to three times a year, children even more. This leads to a great economically loss, since people have to get treatment and usually are not able to come to work during the infection. Further, the standard treatment of influenza and common cold diseases is cost-intensive and shows side effect, which can be severe.

Influenza, also known as flu, is a contagious viral infection which spreads around the world in seasonal epidemics. One distinguishes three virus types, A, B and C. B and C are restricted to humans, while type A extends to mammals and birds.

The World Health Organization, WHO, warns of a global influenza pandemic in the upcoming years. Epidemics and pandemics are mostly caused by influenza viruses of type A. Major genetic changes of the genetic material of influenza viruses have caused three pandemics in the 20^{th} century, the infective agents of which were all of type A.

At present, the avian flu, also a type A virus, represents a particular danger of a pandemic. It has occurred increasingly in recent years, particularly in Southeast Asia. Its spread is aided by wild birds, which serve as resistant carriers of the disease. Experts fear that the avian flu virus could cross with an infective agent of the human flu. In principle, this is possible when pigs or humans are simultaneously infected with the avian flu and an infective agent of the human flu. This could lead to a virus which is highly contagious and deadly for humans, which could result in a global pandemic. Up until now, transmission of the avian flu to humans has only taken place locally. Transmission of the avian flu between humans was, however, not observed.

Vaccination represents the most important means of preventing of a viral sickness. However, in the context of prevention, vaccination depends on the preparation of a vaccine against a certain virus. This requires that the virus must already exist. This, and the long time needed for the development of a vaccine (approximately 4 months), lead to a substantial restriction in its use in a global pandemic. In such a case, the use of vaccines is only ensured by the prior and accompanying use of antiviral agents (WHO Guidelines on the Use of Vaccines and Antivirals during Influenza Pandemics; World Health Organization 2004).

Antiviral agents which are efficacious in treating influenza include amantadine, rimantadine, zanamivir, and oseltamivir und ribavirin. All listed medicaments have side effects which in some cases can be severe. For example, oseltamivir, which is sold under the name Tamiflu®, shows the frequent side effects of nausea, vomiting and stomach pain. Its use is indicated only after 13 years of age, as in some cases severe side effects such as ear infections, pneumonias, infections of the nasal sinuses, bronchitis, swelling of the lymph nodes, and conjunctivitis (Red List, Catalogue of Medication for Germany, 2004) were observed in youths under the age limit.

Antiviral medicaments are efficacious in the prophylaxis of a viral sickness as well as in its treatment. The direct medical cure of a viral sickness has not been successful thus far.

An elderberry extract is known for its effect of shortening the duration of influenza under certain circumstances, without, however, demonstrating any appreciable preventative effect (Zakay-Rones, Z.; Varsano, N.; Zlotnik, M.; Manor, O.; Regev, L.; Schlesinger, M.; Mumcuoglu, M. J. Altern. Complement. Med. 1995, 1 (4), 361-9).

WO-A-99/44578 describes the use of isoquercetin, a natural flavonoid, in medical formulations as a light protection filter and antiviral substance. In particular, isoquercetin, which is for example present in *Ribes nigrum* L., is described as showing antiviral activity against Herpes simplex Type I virus. However, neither the preparation of an extract from *Ribes nigrum* L. nor a particular effect of the antiviral activity of such an extract from *Ribes nigrum* L. has been demonstrated.

Moreover, JP-A-2001-328941 discloses anthocyanins isolated from *Ribes nigrum L.* extracts. Said anthocyanins are described as showing antiviral activity against influenza A or influenza B. However, only pure anthocyanins isolated from extracts of *Ribes nigrum L.* have been studied in view of their activity against human influenza A, the activity of the extract itself has not been examined.

As a preventative measure for the case of an impending pandemic which could be caused by the avian flu, the countries of the world community are counting on antiviral medicaments. For example, the medicament oseltamivir mentioned above (Tamiflu®; Hoffman La Roche) was ordered in significant amounts by some countries as a reserve for the case of a pandemic, although it is feared that the medicament could be quickly exhausted in an emergency. In addition, the immense demand has led to production bottlenecks.

In addition, the use of (known) antiviral agents is increasingly jeopardized by the fact that these are used as broad-spectrum medicaments in animal husbandry. Regardless of international bans, such practices have led for example in China to a resistance of some strains of the avian flu to these agents. In addition to this are the frequent side effects of these agents which in some cases can be severe. Further, in some cases these medicaments are only indicated for certain age groups, such as for example oseltamivir (Tamiflu®), which can only be used after 13 years of age.

Besides influenza, another typical viral infection are common cold diseases. Included among the typical common cold diseases are respiratory tract infections, such as head colds and inflammations of the tonsils and pharynx, as well as coughs and bronchitis. Usually these occur one after the other, but the cold can also remain restricted to the nose, throat or bronchia. Common cold diseases of this kind are also called "viral colds". These are not to be confused with the influenza produced by influenza viruses, which shows a considerably longer and more serious progress of the disease and is, as a rule, associated with fever.

The common cold diseases mentioned are also caused by viruses. Because there are, for example, more than a hundred different types of viruses that can cause a head cold, it will scarcely be possible to develop a vaccine against it. Treatment of head colds or colds in general is therefore targeted at relieving the symptoms. Usually well-tried household remedies are used in these cases. For example, a very congested nose can be helped by the inhalation of hot steam. It allows the swelling in the nasal mucosa to go down and promotes the discharge of the mucous. This can be aided, for example, by the addition of a few drops of tea-tree oil or camomile oil into the hot water. It is also known that routine rinsing of the nose with a saline solution can reduce the susceptibility to head colds.

In addition to the self-help measures, medicines can help to constrict the vessels in the swollen nasal mucosa, leading to a soothing of the nasal mucosa. Nose drops for reducing the swelling of the nasal mucosa should not be used longer than two or three days, however. After this time, it is possible that when the drops are discontinued, the nasal mucosa will swell up all the more, and "rebound swelling" (rhinitis medicamentosa) develops.

Unlike chemical synthetic nasal sprays, phytopharmaceuticals have few side-effects. Even if used over a longer time period, they do not damage the nasal mucosa and do not lead to rhinitis medicamentosa. The sooner phytopharmaceuticals are applied, the more effective they are. They can already be used for support at the first signs of a cold. They also counteract the spread of infection.

For example, often echinacea preparations are taken for common cold diseases, whereby a great number of various medicines in variable phytochemical compositions are on the market. Controlled studies on the efficacy of these phytotherapeutic agents exist only to a limited degree, however, and with contradictory results. Just recently, however, a new study revealed that echinacea does not have the postulated efficacy. The study was conducted with three echinacea preparations with various phytochemical profiles, whereby these preparations were acquired by the extraction of *E.-angustifolia* roots with carbon dioxide, 60% ethanol or 20% ethanol. The total of 437 volunteers with rhinovirus infections who took part in this study received the medicine either as a prophylaxis seven days before exposure to the virus or for treatment at the time of the exposure. The study included a control group that received placebos. There was no significant difference between the three echinacea extracts and the placebo with regard to infection rate, severity of the symptoms, volume of the nasal secretion, leukocyte level, interleukin-8 concentration in the nasal douche water or quantitative virus titres (Deutsches Ärzteblatt 102, Issue 48 from 2 December 2005, page A-3341 / B-2822 / C-2640 and the New England Journal of Medicine, 2005, 353, 341-348).

A further medicine on a botanical basis is an extract from the roots of Pelargonium *reniforme* or *sidoides,* which is marketed under the name Umckaloabo®. Umckaloabo® is traditionally used not only for respiratory tract illnesses, but also for gastrointestinal illnesses. The ingredients determining the efficacy are currently considered to be a number of antibacterial and immunomodulating components, such as coumarins and tannins. It is postulated that the extract develops antibacterial, antiviral and secretolytic effects, whereby the medicine should not be used by pregnant or nursing women, or by patients with liver or kidney diseases or an increased bleeding tendency, because it has not yet been possible to collect sufficient experience in this area. Moreover, Umckaloabo® is quite costly compared to other phytopharmaceuticals.

Other serious diseases are caused by retroviruses.

A retrovirus is an RNA virus which uses the enzyme reverse transcriptase to produce DNA from its RNA genome. Reverse transcriptase is a DNA polymerase enzyme that transcribes single-stranded RNA into single-stranded DNA. Further, reverse transcriptase helps in the formation of a double helix DNA once the RNA has been revers transcribed into a single-strand DNA. The so formed DNA of the virus is then incorporated into the hosts genome by an integrase enzyme and replicated with it. Retroviruses are enveloped viruses belonging to the family of *Retroviridae* and can be divided in endogenous and exogenous retroviruses.

An endogenous retrovirus is present as a genetic element in the chromosomal DNA. It is derived from ancient viral infections in humans, mammals and other vertebrates and passed on to the next generation and remains in the genome. Human endogenous retroviruses are suspected to play a role in some autoimmune diseases, in particular multiple sclerosis.

Exogenous retroviruses are horizontally-transmitted infectious RNA-containing viruses which are transmitted from animal to animal or person to person. Horizontal transmission occurs almost exclusively by body fluids, transmission by smear infections is extremely rare and transmission via air can be excluded. Diseases induced by or associated with exogenous retroviruses are, e. g., feline leukemia or sarcomas, chicken leukemia or sarcomas, mouse leukemia or sarcomas, equine infectious anemia, bovine leukemia, caprine arthritis-encephalitis, human adult T-cell leukemia, human tropic spastic paraparesis, and AIDS.

However, one of the most serious viral infections in humans is an HIV (human immunodeficiency virus) infection. HIV infection in humans has become pandemic. Most HIV infected individuals eventually develop AIDS, a condition in humans in which the immune system begins to fail, leading to life-threatening opportunistic infections. Since 1981 when AIDS was first recognized it has killed more than 25 million people. In 2007 between 30.6 and 36.1 million people were believed to live with HIV, approximately 2.1 million people died and 2.5 million new infections were reported. In Africa, having the highest prevalence of HIV, the average life expectancy is approximately 6.5 years less than it would be without the disease. This leads to a great economical loss and increased poverty.

An HIV infection can be divided into four stages: Primary infection, clinically asymptomatic stage, symptomatic HIV infection and progression from HIV to AIDS.

The first stage of infection, the primary HIV infection, lasts for a few weeks and can include flue-like symptoms. In this stage, the immune system begins to produce HIV antibodies and cytotoxic lymphocytes due to a large amount of HIV in the peripheral blood.

In stage 2, the clinically asymptomatic stage or latency stage, the number of viral particles in the peripheral blood is reduced by strong uimmune defense. Said stage lasts for approximately 10 years and is free from major symptoms. However, HIV is active in the lymph nodes and people remain infectious.

Stage 3 of the infection, the symptomatic HIV infection, characterizes the stage wherein the immune system becomes highly damaged by HIV. The lymph nodes and tissues become damaged due to the years of activity, HIV mutates and becomes more pathogenic, leading to a higher number of T helper cell destruction, and the infected body fails to keep up with replacing the T helper cells that are lost. Due to the decline in T helper cell numbers, cell mediated immunity is lost, and a variety of opportunistic infections appear. This stage eventually leads to stage 4 of the infection, namely the progression from HIV to AIDS.

Vaccination represents the most important means of preventing viral sickness. However, currently no vaccine or cure for HIV infection is available.

Generally, infections caused by retroviruses are treated with antiretroviral drugs. HIV infections are currently treated by highly active antiretroviral therapy (HAART). Said therapy is a combination of at least three different drugs belonging to at least two classes of antiretroviral agents. If only one drug was taken, HIV would become resistant to said drug. Taking several antiretroviral drugs at the same time reduces the rate of resistance development, making treatment more effective in the long term. Currently, more than 20 approved antiretroviral drugs divided into 5 groups are available. Each of these groups attacks HIV in a different way. A first group of antiretroviral agents are nucleoside/nucleotide reverse transcriptase inhibitors (NRTI). Said inhibitors interfere with the reverse transcriptase protein which is required by the virus to make new copies of itself. A second group of compounds are non-nucleoside reverse transcriptase inhibitors (NNRTI), which hinder HIV from replication by inhibiting the reverse transcriptase protein. A third group of antiretroviral agents are protease inhibitors (PI). Said agents inhibit protease, which is required in the HIV replication process. A fourth class of drugs are fusion or entry inhibitors, which prevent HIV from binding to or entering human immune cells. Said drugs are particularly used for patients who are infected with viruses already resistant to common therapies. A fifth class of inhibitors are integrase inhibitors which interfere with integrase. Said enzyme is needed for the virus to insert its genetic material into human cells.

Highly active antiretroviral therapy does not cure the patient, but improves the general health and quality of life of HIV infected patients. The average life expectancy of an HIV infected person now is approximately 32 years from the time of infection. In the absence of HAART, progressions of HIV infection to AIDS usually occurs after 9 to 10 years and the median survival time after developing AIDS is only 9 months. The development of HAART as effective therapy for HIV infection has substantially reduced the death rate in those areas where the drugs are widely available.

The most common drug combination in highly active antiretroviral therapy consists of two NRTIs combined with either an NNRTI or a protease inhibitor. Most commonly, ritonavir is used as the protease inhibitor. An example of an antiretroviral drug combination contains two NRTIs, namely zidovudine and lamivudine, in combination with the NNRTI efavirenz. Commonly used NRTIs are lamivudine, abacavir, zidovudine, stavudine, zalcitabine, didanosine, emtricitabine and tenofovir. Typically used NNRTIs are delavirdine, efavirenz, etravirine and nevirapine. Standard protease inhibitors are amprenavir, fosamprenavir, atazanavir, darunavir, indinavir, lopinavir, ritonavir, nelfinavir, saquinavir and tipranavir. As fusion or entry inhibitors enfuvirtide and maraviroc are normally used. A commonly used integrase inhibitor is raltegravir.

However, sometimes patients show a medication intolerance and high active antiretroviral therapy in some cases causes serious side effects, which can even be life-threatening in rar cases. Further, non-adherence and non-persistence are the major reasons for a failing therapy. Reasons for non-adherence and non-persistence are psychosocial issues, e. g. poor access to medical support, inadequate social support, psychiatric disease and drug abuse. Further, the regimens are very complex, requiring a great number of pills, specific dosing frequency, meal restrictions and other issues. Typical side effects which occur in the therapy are lipodystrophy, dyslipidemia, insulin resistance, an increase in cardiovascular risks and birth defects. Further side effects which occur during treatment with antiretroviral drugs are diarrhoea, nausea, vomiting, rash, hypersensitivity reactions, appetite loss, central nervous system effects, such as dizziness, mood changes, depression, anxiety, and paranoia, fatigue, insomnia, kidney damage, liver damage, pancreas damage, lactic acidosis and nerve damage. Said side effects can have a major impact on health or quality of life.

Moreover, antiretroviral drugs are expensive and the majority of the infected individuals does not have excess to medications and treatments for HIV and AIDS. For example, fusion and entry inhibitors, as well as integrase inhibitors are only available in resource-rich countries.

US-A-2005/0266104 relates to the extraction and purification of flavonoid compounds from plant material. Tabart et al., Food Chemistry 2007, 105, 1268 to 1275 studied the extraction of phenolics and antioxidants from black currant leaves. Knox et al., Acta virologica 2001, 45, 209 to 215 describe the antiviral activity of *Ribes nigrum* fruits against influenza A and B. Tabart et al., J. Agric. Food Chem. 2006, 54, 6271 to 6276 describe the antioxidant properties of flavonols isolated from black currant. Knox et al., Phytotherapy Research 2003, 17, 120 to 122 studied an anti-influenza virus activity of crude extracts of *Ribes nigrum.* Suzutani et al., Phytotherapy Research 2003, 17, 609 to 613 describe an anti-herpes activity of an extract of *Ribes nigrum.* Middleton et al., Pharmacological Reviews 2000, 52, 673 to 751 describe the antiviral effect of naturally occurring flavonoids. Brinkworth et al., Biochemical and Biophysical Research Communications 1992, 188 (2), 631 to 637 studied flavonoid compounds having antiviral activity against HIV. Korovka et al., FSTA Abstract 77-3-05-a0336 determined the ascorbic acid content in wild plants.

The object of the present invention is therefore to provide the use of an antiviral composition for the prophylaxis and/or treatment of viral infections, which can be economically prepared and which causes no side-effects at all or only minor side-effects when administered.

This object is achieved by a composition for the use in the prophylaxis and/or treatment of viral infections, wherein the composition comprises an aqueous extract from at least one of the aerial parts of plants of the genus Ribes, wherein the aerial parts are selected from the group consisting of leaves and twigs.

### Description of the figures

Figure 1 shows the results of the antiviral activity of an extract of the twigs and leaves of *Ribes nigrum* against Influenza A Virus A/Puerto-Rico/8/34 (H1N1) (PR8) (human).

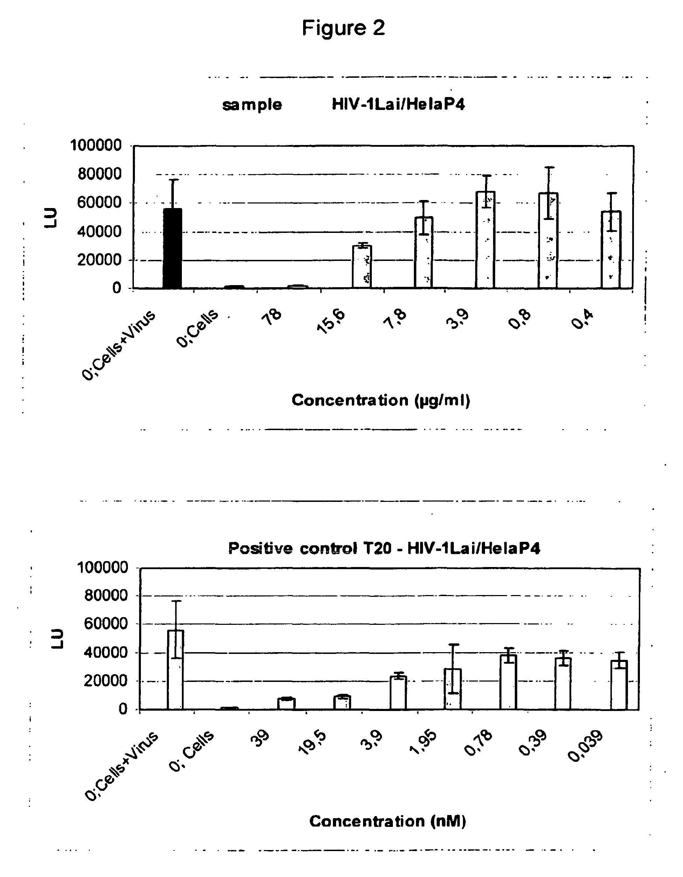

Figure 2 shows the results of the antiviral activity of an extract of the twigs and leaves of *Ribes nigrum* and of T20 against HIV-1.

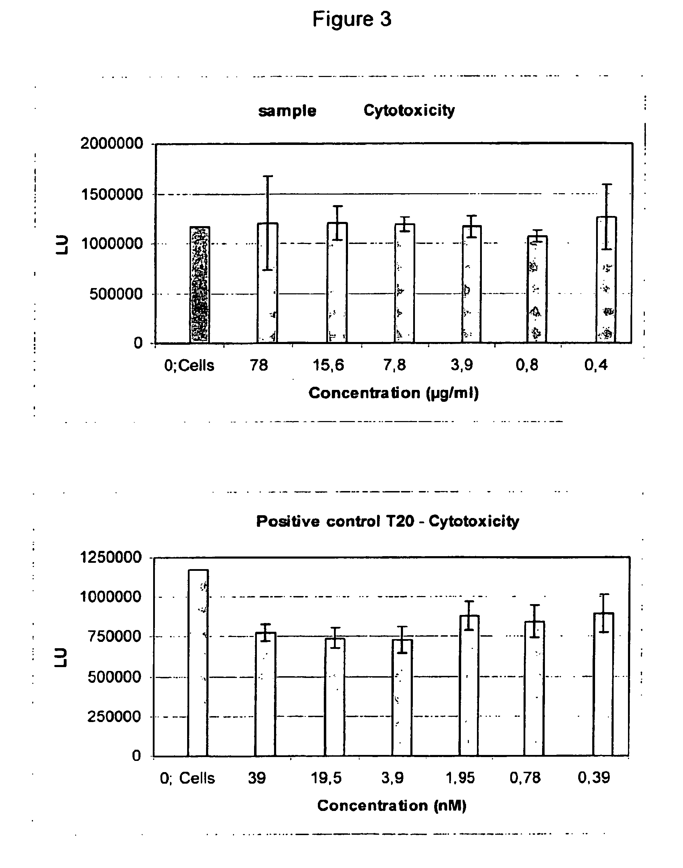

Figure 3 shows the results of the cytotoxicity assay of of an extract of the twigs and leaves of *Ribes nigrum* and T20.

### Detailed description of the invention

The present invention relates to a composition for the use in the prophylaxis and/or treatment of viral infections, wherein the composition comprises an aqueous extract from at least one of the aerial parts of plants of the genus Ribes, wherein the aerial parts are selected from the group consisting of leaves and twigs.

In a preferred embodiment, in combination with any one of the embodiments listed above or below, the viral infection comprises a common cold disease.

More preferably, the common cold disease comprises a primary infection, caused by rhinoviruses, adenoviruses or coronaviruses.

In a further preferred embodiment, in combination with any one of the embodiments listed above or below, the composition is used for the treatment of head colds.

In another preferred embodiment, in combination with any one of the embodiments listed above or below, the viral infection comprises influenza. More preferably, the influenza is avian flu.

In a further preferred embodiment, in combination with any one of the embodiments listed above or below, the viral infection is caused by retroviruses, more preferably by lentiviruses. In particular, the viral infection is caused by HIV-1 and/or HIV-2.

In a preferred embodiment, in combination with any one of the embodiments listed above or below, the plant is *Ribes nigrum L..*

In another preferred embodiment, in combination with any one of the embodiments listed above or below, the plant is *Ribes rubrum L..*

In a preferred embodiment, in combination with any one of the embodiments listed above or below, the composition further comprises an extract of the fruits of the plants of the genus Ribes.

In a preferred embodiment, in combination with any one of the embodiments listed above or below, the composition is in liquid, dry or semi-solid form.

In a preferred embodiment, in combination with any one of the embodiments listed above or below, the composition is administered orally, intranasally or topically.

In an alternative preferred embodiment, the composition is present as a nasal agent, inhalation mixture, aerosol or room spray.

The composition may also preferably be present in the form of a tablet, coated tablet, effervescent tablet, capsule, powder, granulate, sugar-coated tablet, ointment, gel, cream, gargling solution or plant juice.

The present invention relates to the use of an aqueous extract from at least one of the aerial parts of plants of the genus Ribes, wherein the aerial parts are selected from the group consisting of leaves and twigs for the preparation of a medicament for the prophylaxis and/or treatment of viral infections.

In the present invention the term "aerial parts of a plant" refers to leaves and twigs.

In the present invention, the term "twig" refers to a small shoot or branch having a diameter of 3 cm at most. Preferably, the diameter of the twigs is up to 1 cm.

According to the invention, the term "aqueous extract" is used representatively for all products that are obtained from a herbal subject by means of an extraction with water, such as with maceration or percolation.

In the present invention, the term "prophylaxis" refers to a procedure to prevent a disease. Prophylactic measures can be divided into primary prophylaxis (prevention of the development of a disease) and secondary prophylaxis (protection against worsening when a disease has already developed).

Influenza infective agents are viruses of the type A, B and C. Seasonally occurring influenza in humans is caused by the influenza type A virus with the subtypes H1, H2, and H3, as well as by the influenza type B virus. The avian flu is primarily caused by the subtypes H5, H7, and H9.

The described extract is particularly suited for the prophylaxis and/or treatment of the avian flu. In particular, the extract can be used for the prophylaxis and/or treatment of avian flu caused by the subtypes H5 and H7.

Understood as common cold diseases for the purposes of the invention are inflammations of the respiratory tract, meaning, as a rule, the nose, pharynx, larynx, trachea and bronchia. The terms "common cold diseases" and "viral colds" are used synonymously in this case. A viral cold is distinguished from influenza in that the latter is caused only by influenza viruses.

A viral cold, on the other hand, is usually brought about by adenoviruses, coronaviruses and/or rhinoviruses.

Adenoviruses (Adenoviridae) belong to the family of the non-enveloped cubic DNA viruses and have a diameter of 60 to 90 nm. The genome consists of a linear double-stranded DNA approximately 36 kb long. One differentiates approximately 50 immunologically distinct types of adenoviruses, with approximately 35 human pathogenic types in the sub-genera A - F. The Adenoviridae family is divided into the genera Mastadenoviruses, which can infect mammals, and Aviadenoviruses, which are endemic in various bird species. Adenoviruses are characterised by unusual stability in the face of chemical and physical effects and tolerate the most adverse pH levels, which allows them a comparatively long survival time outside the host body.

Adenoviruses primarily cause illnesses of the respiratory tract. Depending on the particular serotype, however, a number of other illnesses can also be caused, for example, gastroenteritis, conjunctivitis, cystitis, pharyngitis or diarrhoeas. The symptoms of the respiratory tract illness caused by adenoviruses range from the common cold to bronchitis to pneumonia. In the case of patients with weakened immune systems, there is special susceptibility to serious complications from the adenovirus infections, such as ARDS (Acute Respiratory Distress Syndrome), for example. Furthermore, it is suspected that there is a correlation between the virus type Ad-36 and obesity in humans.

Coronaviruses, which belong to the genus Coronaviridae, generally cause mild illnesses of the upper respiratory tract in humans, seldom gastroenteritis and the Serious Acute Respiratory Syndrome (SARS), caused by the SARS-associated coronavirus SARS-CoV. The coronaviruses are classified in the family of the enveloped pleomorphic RNA viruses and have a diameter of 70 to 160 nm. They have a single-stranded positive-sense RNA with a length of from 20 to 30 kb. The Coronaviridae genus is divided into three genera: the coronaviruses, the arteriviruses and the toroviruses. Of these, only the coronaviruses comprise human pathogenic viruses. The transmission of the viruses takes place through droplet infection (aerogenic), as a dirt or smear infection (faecal-oral) or even through simple contact (mechanical) with an infected person. Younger infected organisms in this case can become more seriously ill than older ones. Coronaviruses cause between 15 to 30% of the common cold diseases in humans with a slight fever, head cold, cough and sore throat.

An acute or chronic irritation of the nasal mucosa with the symptoms of itching, sneezing, secretion and congestion caused by infectious, allergic and non-allergic mechanisms is called rhinitis, nasal catarrh, coryza or colloquially, head cold. The pathogen is usually a genus of the picornavirus - the rhinovirus. The infection with rhinoviruses takes place through direct transmission, e.g., via contaminated hands or also via droplet infection.

More than 115 serotypes of this genus have been identified until now. Rhinoviruses have a single-stranded, positive-sense RNA (messenger RNA) with a length of from 7.2 to 8.5 kb. These are naked viruses with an icosahedral structure and a diameter of from 24 to 30 nm. The 10 to 15 nm-thick protein envelope (capsid) surrounding the RNA consists of 60 symmetrically arranged subunits, which are called protomers. Each protomer consists of the four capsid proteins VP1, VP2, VP3 and VP4. The multiple number of protomers is considered the cause of the antigenic versatility of the rhinoviruses.

As already mentioned, common cold diseases are usually caused by adenoviruses, coronaviruses and/or rhinoviruses. Depending on the type of infection virus, cold complaints such as head cold, coughing, hoarseness, sore throat, for example, caused by inflammation of the tonsils and pharynx, joint pain and head ache, chills, slight fever and exhaustion can occur. For the purposes of the invention, bronchitis and bronchial pneumonia are also counted as common cold diseases.

Of these common cold diseases, a head cold in the winter months occurs most frequently. It is caused by an infection with rhinoviruses, or, less frequently, with adenoviruses. The described composition or preparation is preferably used for the prophylaxis and/or treatment of head colds, in particular in the prophylaxis and/or treatment of head colds caused by rhinoviruses.

Furthermore, bacterial infections, which "set up" on the already existing virus infection, can occur with common cold diseases. Infections of this kind are called secondary bacterial infections or bacterial superinfections. The composition for use according to the invention may also be used for the prophylaxis and/or treatment of these secondary bacterial infections.

Virions of retroviruses consist of enveloped particles having a particle diameter of about 100 nm and also contain two identical single-stranded RNA molecules of 7 to 10 kb in length. The main components of retroviruses are
- an envelope composed of a lipid bilayer obtained from the host plasma membrane,
- dimer RNA having a cap at 5' end and polyadenyl at 3' end, and
- proteins containing gag proteins as major components of the viral capsid, protease functioning in proteolytic cleavages during virion maturation, pol proteins responsible for the synthesis of viral DNA and integration into host DNA after infection, and env proteins playing a role in association and entry of virion into the host cell.

HIV is a virus belonging to the family of *Retroviridae* and the genus of lentivirus. It is roughly spherical having a diameter of about 120 nm and is composed of two copies of positive single-stranded RNA, which is bound to nucleocapsid proteins and enzymes required for the development of the virion (e. g. reverse transcriptase, proteases, ribonuclease and integrase). The capsid is surrounded by a matrix composed of viral protein, which is further surrounded by the viral envelope composed of two layers of phospholipids from the membrane of a human cell. In said envelope further proteins from the host cell and copies of a complex HIV protein are embedded. The protein consists of a cap made of three molecules of glycoprotein and a stem that anchors the structure into the viral envelope. The glycoprotein enables the virus to attach and fuse with target cells to start the infectious cycle.

HIV infects cells in the immune system and the central nervous system. In particular, HIV enters macrophages and T helper cells, in particular CD4+ T cells, by adsorption of the glycoprotein to receptors on the target cell, fusion of the viral envelope with the cell membrane and release of the HIV capsid into the cell. After penetration into the cell, HIV produces new copies of itself which continue to infect other cells. Thus, HIV infection finally leads to a reduction in the number of T helper cells through three main mechansims by direct viral killing of infected cells, increased rate of apoptosis in infected cells and killing of infected CD4+ T cells by CD8 cytotoxic lymphocytes that recognize infected cells. If the level of CD4+ T cells declines below a critical level, cell-mediated immunity is lost and the body becomes more susceptible to opportunistic infections.

HIV can be further divided into HIV-1 and HIV-2. For HIV-1, the subtypes A to J exist, the most common subtypes are 1A, 1B, 1C and 1D. HIV-2 can be divided in subtypes A to E. It is less pathogenic than HIV-1.

Besides HIV, another human retrovirus is the human T-lymphotropic virus, in particular human T-lymphotropic virus Type 1 (HTLV-1). HTLV-1 is a human RNA retrovirus that causes T-cell leukemia and T-cell lymphoma and may also be involved in demyelinating diseases, such as tropical spastic paraparesis.

Further retroviruses include Avian leukosis virus, Rous sarcome virus, Mouse mammary tumor virus, Murin leukemia virus, Feline leukemia virus, Bovine leukemia virus, Walley dermal sarcoma virus, Simian and Feline immunodeficency virus, Equine infectious anemia virus and Simian foamy virus causing diseases such as feline leukemia or sarcomas, chicken leukemia or sarcomas, mouse leukemia or sarcomas, equine infectious anemia, bovine leukemia, and caprine arthritis-encephalitis.

According to the invention, the Ribes plant is used for producing a composition for use in the prophylaxis and/or treatment of viral infections.

Ribes is a genus covering about 150 species of flowering plants, which are native throughout the temperate regions of the Northern Hemisphere, and in the mountain ranges of Central and South America. The genus of Ribes includes the currants and gooseberries.

Of particular interest in the present invention are:

### Ribes nigrum L. (R. nigrum L.)

### Ribes rubrum L. (R. rubrum L.)

*R. nigrum* L. and *R. rubrum L.* contain flavonoids, terpenoids and essential oils in different concentrations. For example, the concentration may differ with respect to the leaves and fruits of the plant Ribes. Thus, in the following, the leaves and fruits are considered separately.

The scientific term *"Ribes nigri folium (R. nigri folium*)" refers to the leaves of *R*. *nigrum* L., whereas the term *"Ribes nigri fructus (R. nigri fructus*)*"* refers to the fruits of *R. nigrum L..* Accordingly, *Ribes rubri folium (R. rubri folium)* refers to the leaves of *R. rubrum* L., and *Ribes rubri fructus (R. rubri fructus)* to the fruits of *R. rubrum* L*..*

Flavonoids basically consist of two aromatic and one oxygenated heterocyclic ring. Using structural differences on the O-heterocyclic ring, the flavonoids can be divided into the following six groups: flavonols, flavanols, flavanones, flavones, anthocyanins and isoflavanoids. Some of the components detected in *R. nigrum* L. are flavonols, such as quercetin and myricetin and their glycosides, as well as dimers or oligomers of proanthocyanidins.

*R. nigri folium* comprises traces of essential oils, flavonolgylcoside and proanthocyanidine. *R. nigri fructus* contains anthocyanidins and flavonolglycosides, in particular isoquecitrin, myricetin-D-glucopyranosid and rutosin. Further components of *R. nigri fructus* are fruit acids, such as citric acid, isocitric acid and malic acid, hydroxycinammic acid derivatives, Vitamin C and in the seeds γ-linolic acid.

*R. rubri folium* contains flavonolglycosides, such as astragalin and isoquercetin, porcyanidines and catechin derivatives. *R. rubri fructus* contains Vitamin C, fruit acids, pectins, procyanidines and tannins. The seeds comprise γ-linolic acid [Hager's Handbuch der pharmazeutischen Praxis, Drogen P-Z, 5., vollständig neubearbeitete Auflage, Springer-Verlag, 1993, Seite 466-474].

The composition used according to the invention is produced from at least one of the aerial parts of the plants selected from the group of leaves and twigs. Preferably, the aerial shoots of the plant that grow back in the same year are used. In general, all elements of the aerial part of the plant, such as leaves, twigs, blossoms, fruits and seeds can be used. Preferably, the twigs are used with leaves and blossoms.

In another preferred embodiment, in combination with any one of the embodiments listed above or below, an extract from the fruits is added to the composition produced from the leaves and twigs.

The parts of the plant, including leaves, twigs and fruits can be either dried or pressed out directly after the harvest, meaning in the raw state, after being broken up, where appropriate, in order to produce a juice from the pressing.

In a further embodiment, in combination with any one of the embodiments listed above or below, the plant parts are submitted in the raw state to an extraction with water, such as a maceration or percolation, for example. Alternatively, the plant parts can also be dried and/or subsequently broken into small pieces in a suitable manner before the extraction, by means of rubbing or cutting them, for example.

In a preferred embodiment, in combination with any one of the embodiments listed above or below, *R. nigri folium* and/or *R. rubri folium* are used for the preparation of the aqueous extract of the present invention. In another preferred embodiment, in combination with any one of the embodiments listed above or below, *R. nigri folium* and *R. nigri fructus* are used for the preparation of the aqueous extract. In a further preferred embodiment, in combination with any one of the embodiments listed above or below, R. *rubri folium* and R. *rubri fructus* is used. In another preferred embodiment, in combination with any one of the embodiments listed above or below, R. *nigri folium* and R. *rubri fructus* is used for the preparation of the aqueous extract. In another preferred embodiment, in combination with any one of the embodiments listed above or below, R. *rubri folium* and *R. nigri fructus* are used for the preparation of the aqueous extract.

In the present invention, the composition comprises an aqueous extract from the Ribes plant. Generally, an extraction of the plant parts including leaves, twigs and fruits with coates takes place.

The extraction is normally carried out at temperatures of 25° C to, where applicable, as high as the boiling point of water. Preferred is an extraction at 95 to 100 °C.

The extraction is normally carried out for 2 to 8 h. Preferably, the extraction is carried out for 3 to 6 h, more preferably for 4 to 5 h.

In order to achieve the highest possible yield, the plant material can be extracted a number of time. Preferably, the extraction is repeated 2 to 6 times, more preferably 3 times.

As a result of the aqueous extraction, a raw product is obtained, which can be used in this form for producing a composition for the prophylaxis and/or treatment of viral infections.

A maceration procedure is normally performed for five to nine days, preferably for seven days, at room temperature with a mixture of water and ethanol, by pouring the solvent mixture over the plant elements and letting this stand for the period of time mentioned.

According to the invention, a percolation of the plant parts is normally achieved by treating the parts with water at 95 to 100° C for four to five hours by conducting the water through the plant parts.

The crude product obtained from an extraction with water, such as a maceration or percolation, can also be concentrated and/or dried and/or further processed before use. The further processing can, for example, include cleaning steps known to the person skilled in the art, such as centrifugation, filtration and decanting, in order to remove suspended materials from the extract. Chromatography, such as column chromatography, gas chromatography or HPLC or steam distillation may also be used for purification. In a preferred embodiment the crude product is used without further purification steps.

An aqueous extract obtained in this way can subsequently be further processed into a dry extract. To produce the dry extract, the solvent can be withdrawn from the liquid raw extract, the concentrated extract or the cleaned extract by, for example, spray drying, freeze drying or vacuum drying.

The composition from the Ribes plant can be used for the prophylaxis and/or treatment of viral infections in each of the forms described above.

The composition is preferably used for the prophylaxis and/or treatment of common cold diseases that are caused by rhinoviruses, adenoviruses or coronaviruses.

In a preferred embodiment, in combination with any one of the embodiments listed above or below, the aqueous extract of *R. nigrum* L. is used for the prophylaxis and/or treatment of cold diseases caused by rhinoviruses. In particular, said extract is an extract of *R. nigri folium.*

In another preferred embodiment, in combination with any one of the embodiments listed above or below, an extract of *R. nigrum* L. is used for the prophylaxis and/or treatment of cold diseases caused by adenoviruses. In particular, said extract is an extract of *R. nigri folium.*

In a further preferred embodiment, in combination with any one of the embodiments listed above or below, the aqueous extract of *R. nigrum* L. is used for the prophylaxis and/or treatment of cold diseases caused by coronaviruses. More preferably, said extract is an extract of *R*. *nigri folium.*

The described extract is further used for the prophylaxis and/or treatment of influenza A and B. In a preferred embodiment, in combination with any one of the embodiments listed above or below, the extract is suited for the prophylaxis and/or treatment of the avian flu. In particular, the extract can be used for the prophylaxis and/or treatment of avian flu caused by the subtype H7.

Preferably, an extract of *R. nigrum* L. is used for the prophylaxis and/or treatment of influenza A and B, more preferably an extract of *R. nigri folium* is used.

In another preferred embodiment, the composition from the Ribes plant can be used for the prophylaxis and/or treatment of viral infections caused by retroviruses in each of the forms described above. The composition is preferably used for the prophylaxis and/or treatment of viral infections that are caused by lentiviruses, in particular HIV-1 and/or HIV-2.

In a further preferred embodiment, in combination with any one of the embodiments listed above or below, an extract of *R. nigrum* L. is used for the prophylaxis and/or treatment of viral infections caused by retroviruses. In particular, said extract is an extract of *R. nigri folium.* In particular, the extract can be used for the prophylaxis and/or treatment of viral infections caused by HIV-1 and/or HIV-2.

The composition can therefore be administered as a medicine. In addition to therapeutic use, the composition is also suitable for non-therapeutic prophylaxis and/or treatment of common cold diseases.

The composition can be applied in each of the application forms familiar to the person skilled in the art for both medical and non-medical use, e.g., as tablets, coated tablets, effervescent tablets, capsules, powders, granulates, sugar-coated tablets, ointments, creams, gels, solutions or sprays. Preferably, the composition is applied as a nasal spray.

In galenic and other application forms, the composition can be processed with the customary galenic aids, such as tablet bonders, filling agents, preservative agents, tablet-opening agents, flow regulation agents, softening agents, wetting agents, dispersing agents, emulsifying agents, solvents, retarding agents, anti-oxidative agents, consistency regulators, penetration improvers and/or propellant gases.

Further elements, such as vitamins and minerals, can be added to the composition.

The composition can, for example, also be added to animal feed or foodstuffs, such as drinks. In the form of an extract, the composition itself can also be infused as tea. It is also possible, however, for hot water to be poured directly over the plant parts, for example, the leaves of the Ribes plants, for tea preparation. Furthermore, the composition can be a constituent of food supplements, whose ingestion in the winter months can contribute to strengthening the body's defences and consequently to preventing a viral infection.

In a further embodiment, in combination with any one of the embodiments listed above or below, the composition can be used according to the invention as a solution, in particular, a gargling solution, for the prophylaxis and/or treatment of common cold diseases, in particular of inflammations in the mouth and pharynx.

The composition can also be used mixed with constituents of other plants, in which case the constituents are preferably in the form of plant extracts. Preferably constituents of plants or plant extracts with a similar or synergetic effect are used. Examples are plants of the genus Cistus, in particular Cistus incanus.

The concentration of the composition in the application form varies, depending on the type of application. As a rule, the quantity of the composition amounts to between 0.5 and 1,000 mg per dosing unit for solid application forms. Preferably the quantity of the composition amounts to between 1 and 500 mg per unit. In liquid application forms, the composition can be in a concentration of 1 µg/ml to 100 mg/ml, preferably from 25 µg/ml to 50 mg/ml. In the case of semi-solid application forms, the content of the composition amounts to 1 to 90% by weight, preferably 5 to 75 % by weight.

In a preferred embodiment, in combination with any one of the embodiments listed above or below, the composition is administered in the form of a tablet. It is preferable for the composition be in the form of an extract in this case. Most especially preferred, the composition is in the form of a dry extract.

In a further preferred embodiment, in combination with any one of the embodiments listed above or below, the composition is administered in the form of emulsions, ointments, gels or creams for topical application. In this case, the composition is preferably used in the form of an extract in which the active substances are withdrawn from the plant by means of extraction with a fat, wax or oil. It is furthermore preferred for this extract to be further processed into a dry extract, which is subsequently mixed with or dissolved in a fat, wax or oil.

In a preferred embodiment, in combination with any one of the embodiments listed above or below, the composition is in the form of an aerosol or room spray. Preferably a liquid or solid extract of Ribes is used for this. In addition to the extract, the aerosol or room spray can also contain pharmaceutically harmless substances, carrier media and auxiliary agents. The aerosol or room spay can be used for disinfecting objects and rooms with which viruses come into contact or could potentially come into contact, particularly means of transport of all types in which people, animals and/or foodstuffs are transported. For example, an airplane can be sprayed with the aerosol according to the invention or with the room spray according to the invention before takeoff, in order to prevent the spread of the viruses and consequently to minimise the risk of infection for people. The aerosol or room spray can also be sprayed in the presence of people, e.g., in waiting rooms, because it does not cause any toxic effects whatsoever in people.

In a further preferred embodiment, in combination with any one of the embodiments listed above or below, the composition can also be administered as a nasal agent or as an inhalation solution. The nasal agent can be used as a nasal spray or as a nasal gel. For administration, various applicators and dispersion systems can be used.

The use of Ribes according to the invention is not restricted to people, but instead is also possible for animals, particularly mammals, such as pets or livestock.

The following examples explain the invention.

A Ribes extract was tested with respect to its cell toxicity and cell viability, as well as its antiviral activity against rhinoviruses, antiviral activity against influenza A and B and antiviral activity against HIV.

Human rhinovirus type 14 served as the virus isolate in the case of testing antiviral activity against rhinovirus.

Influenza A Virus A/Bratislava/79 (H7N7) (FPV) (avian), Influenza A Virus A/Mallard/Bavarian/1/2006 (H5N1) (avian) as well as the Influenza A Virus A/Puerto-Rico/8/34 (H1N1) (PR8) (human) served as virus isolates in the case of testing antiviral activity against influenza. Madin-Darby canine kidney (MDCK) cells and A549 cells served as host cell lines.

To determine the characteristics of the extract, the following examination methods were used.

Further, the toxic concentration of the extract was determined.

MDCK II canine kidney epithelial cells were treated for 30 minutes with different concentrations of the extract (95 to 950 µg/ml). Subsequently, the cells were treated for 48 hours with the same concentrations of the extract.

### Examination of the cytopathological effect (CPE)

In a first test, MDCK II dog kidney epithelial cells were infected with the influenza virus strains Influenza A Virus A/Mallard/Bavarian/1/2006 (H5N1) and A/FPV/Bratislava/79 (H7N7). The infected cells were subsequently treated with different concentrations of the extract (95, 190, 285, 380, 475, 570, 665, 760, 855 und 950 µg/ml).

In a second test, the cells were pre-treated for 30 minutes with different concentrations of the extract (95 to 950 µg/ml) and were subsequently infected with the influenza virus strains Influenza A Virus A/Mallard/Bavarian/1/2006 (H5N1) and A/FPV/Bratislava/79 (H7N7). The infected cells were subsequently treated with different concentrations of the extract (95, 190, 285, 380, 475, 570, 665, 760, 855 und 950 µg/ml).

In a third test, the virus-containing infection solution was pre-treated with different concentrations of the extract (95 to 950 µg/ml) for 30 minutes. The cells were pre-treated for 30 minutes with different concentrations of the extract (95 to 950 µg/ml) and were subsequently infected with the pre-treated influenza virus strains Influenza A Virus A/Mallard/Bavarian/1/2006 (H5N1) and A/FPV/Bratislava/79 (H7N7). The infected cells were subsequently treated with different concentrations of the extract (95, 190, 285, 380, 475, 570, 665, 760, 855 und 950 µg/ml).

The effective concentration (EC50) for the inhibition of 50% of the CPE was determined.

### Example

### Preparation of an extract from Ribes nigrum L.

The twigs and leaves are used for extraction. The plant material is dried at room temperature outdoors in the shade, down to a residual water content of a maximum of 10%. Subsequently, the plant parts are cut to a size of ≤ 8 mm.

The cut plant parts are submitted to percolation at 95 to 100° C with ten times the quantity of purified water Ph.Eur. for 4 to 5 hours. The solution produced is concentrated to 18 to 19% of the original volume by means of a plate evaporator at a steam temperature of 75 to 80° C. The content of the dry substance amounts to approximately 45%.

Using an evaporator with agitator, the content of the dry substance is increased to 50 to 51% by means of heating the extract for four hours at 110 to 114° C at a reduced pressure (0.6 bar).

Finally, vacuum belt drying at 16 mbar with descending temperature gradients (140° C, 120° C, 90° C, 20° C) is carried out. The content of the dry substance amounts to >92% with an overall yield of the dry extract of 22 to 25%. The extract is subsequently ground. The stock solution described in the preceding is then produced from this extract.

The concentration at which 50% of the MDCK II cells were dead (TC50) was 8512 ± 11,40 µg/ml.

It is therefore concluded, that the extract is not toxic.

### Examinations of antiviral activity

### Human Influenza viruses

For the investigations of the antiviral activity, A549 lung epithelial cells were pre-treated for 30 minutes with 50 µg/ml of the extract and were subsequently infected with the influenza virus strain A/PR8/34 (H1N1), which has also been pre-treated for 30 minutes with 50 µg/ml of the extract. After infection, the cells were treated again for 30 minutes with 50 µg/ml of the extract. The medium supernatants were isolated and were investigated in plaque assays for newly formed influenza viruses.

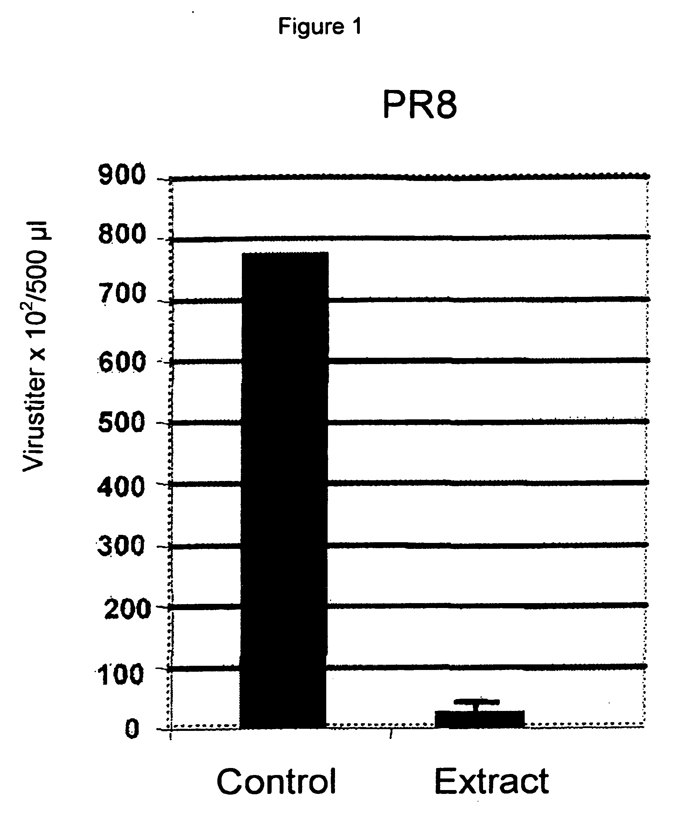

The result is shown in Figure 1.

As can be taken from Figure 1, a reduction of the virus titer by more than one order of magnitude was observed.

A strong inhibitory effect on the virus propagation of different influenza viruses is observed in the host cell lines.

### Examination of the cytopathological effect (CPE) induced bv avian Influenza viruses

The first test (post-treatment of the cells) showed an EC50 value of the extract of 390,93 ± 85,5 µg/ml for Influenza A Virus A/FPV/Bratislava/79 (H7N7) and of 165,3 ± 8,55 µg/ml for Influenza A Virus A/Mallard/Bavarian/1/2006 (H5N1).

The second test (pre- and post-treatment of the cells) showed an EC50 value of the extract of 278,35 ± 48,45 µg/ml for Influenza A Virus A/FPV/Bratislava/79 (H7N7) and of 83,6 ± 2,85 µg/ml for Influenza A Virus A/Mallard/Bavarian/1/2006 (H5N1).

The third test (pre- and post-treatment of the cells and additionally pre-treatment of the virus solution) showed an EC50 value of the extract of 46,55 ± 6,65 µg/ml for Influenza A Virus A/FPV/Bratislava/79 (H7N7) and of 13,3 ± 1,9 µg/ml for Influenza A Virus A/Mallard/Bavarian/1/2006 (H5N1).

Further, no inhibition of the Influenza A viral Neuraminidase was observed.

In the result, it could be seen that the Ribes extract was able to inhibit the infection caused by highly pathogenic avian Influenza viruses. In the concentration used, the extract had no detectable damaging influences on the cells whatsoever. This proves an inhibitory effect of the Ribes extracts on the infectivity of influenza.

### Rhinoviruses culture

Four T175 flasks with 80% confluent HeLa cells were inoculated with human rhinovirus type 14 (HRV) and incubated at 33° C for one week. To do this, 50 µl HRV14 (virus titre 10⁸/ml TCID (Tissue Culture Infectious Dose)) in 16 ml infection medium (DMEM (Dulbecco's Modified Eagle Medium), 2% FCS (Foetal Calf Serum), 10-20 mM MgCl₂) are mixed and 4 ml added to each T175. Each T175 is then filled up with 10 ml infection medium, so that 14 ml infection medium is in each T175. As soon as 70% of the adherent cells dissolve, the virus is harvested.

### Purification of the rhinoviruses

The virus supernatant is first centrifuged for 30 minutes at 3,000 rpm in order to remove the cell pellet. In the ultracentrifuge, the virus supernatant is centrifuged at 35,000 rpm (rotor type SW41 Ti in Beckman polyallomer tubes) at 4° C for three hours on sucrose cushions (1.5 ml sucrose 65% in water, 300 µl 10xPBS (Phosphate-Buffered Saline), 1.2 ml water) and the pellet is incorporated into 100 µl infection medium. Further virus concentration takes place with a 100 kDa cut-off filter (Centricon YM100) at 3,300 rpm for one hour at 4° C. The retentate contains the purified virus concentrate; the filtrate is discarded.

In order to test the effect of the Ribes extract on the infectivity of HRV14, the Ribes extract was added to the infection medium or the viruses were additionally pre-treated with the Ribes extract.

In the result, it could be seen that the Ribes extract was able to prevent the infection and therefore the destruction of the cell layer in both the infection medium and after additional pre-incubation of the viruses in all of the batches conducted. The Ribes extract had no detectable damaging influences on the cells whatsoever. This proves an inhibitory effect of the Ribes extracts on the infectivity of rhinoviruses.

### In vitro testing of substances of antiviral activity in the HeLa-P4 assay

### Principle of the assay

HeLa-P4 cells (from NIH AIDS Research and Reference Program) are a cell line, which was transfected with genes for the human CD4- and CCR5 receptor and which are therefore infectable with HIV-1 (the CXCR4-receptor is expressed on the cells). For quantification of the HIV infection, the cells carry a reporter gene, i.e. β-Galactosidase gen, which is under control of the HIV-1 promoter. After infection, the Tat-protein of HIV-1 transactivates the reporter gene and the degree of infection can be quantified by measurement of the enzyme activity in the lysate of the cells. An inhibition of HIV-1 infection results in a lower β-Galactosidase activity with respect to the infection with viruses without substance addition. The background is defined by measuring the enzyme activity in cells, to which no virus was added. Since in principle a lower infection may also be based on toxic effects of the substances to the cells, in addition to said assay a cytotoxicity assay is also carried out.

### Experimental

The test substances are prepared by adding 1 mg of each sample in an Eppendorf vial and dissolving the sample in 1 ml of phosphate buffered saline (PBS) at 60° C under vortexing. The solution is filtered under sterile conditions and placed in a new Eppendorf vial. As a positive control, the T20-peptide (a known HIV-1 entry inhibitor; Enfuvirtide) is used. Dilution series of the test compound as well as of T20 in cell culture medium were prepared, wherein the the samples have concentrations of 78, 15.6, 7.8, 3.9, 0.8, 0.4 µg/ml, and the positive control T20 is used in concentrations of 39, 19.5, 3.9, 1.95, 0.78, 0.39, 0.039 nM. Each of the substance concentrations was tested in triplicate.

Day 1: Plating of 1.5 x 10⁴ HeLa-P4 cells in 100 µl of medium (DMEM, 10% FCS, 2% L-glutamine, 1% penicillin/streptomycin, 500 µg/ml geniticin, 1 µg/ml puromycin) per each well of a 96-well microtiter plate, incubation by 37 °C overnight.

Day 2: In an L3 laboratory, 78 µl of each of the substance dilutions (78, 15.6, 7.8, 3.9, 0.8, 0.4 µg/ml) is pre-incubated with 22 µl of the HIV-1 Lai virus at 37 °C for 30 minutes, the T20 dilutions (39, 19.5, 3.9, 1.95, 0.78, 0.39, 0.039 nM) are treated in the same manner. The medium is removed from the HeLa-P4 cells, the cells are washed with 100 µl of PBS and the substance-virus mixtures are added to the cells. The cells are incubated for 2 h at 37 °C, afterwards the supernatant is removed, it is washed with PBS and 100 µl of fresh medium is added, and the cells are incubated for 2 days at 37 °C.

Day 4: The supernatant is removed, the cells are washed with 100 µl of PBS and lysed in 50 µl of lysis buffer per each well (2.5 ml glycerol; 1.25 ml MES-Tris, 25 µl 1 M DTT, 250 µl Triton X100, H₂O ad 25 ml) for 10 minutes on ice and removed at -80° C. The plates are sprayed on the outside with Biguanid for transferring out of the L3 laboratory. In a white micro titer plate, 34 µl of reaction buffer (15 µl 1 M MgCl₂, 3 ml 0.5 M NaH₂PO₄/Na₂HPO₄, 150 µl Galacton 100x, ad 15 ml H₂O) is placed in each well and 20 µl of cell lysate is added. The micro titer plate is shaken for 45 to 60 minutes in the dark and subsequently after addition of 25 µl of amplifier (80 µl 10 M NaOH, 400 µl 10x Emerald (Applied Biosystems), ad 4 ml H₂O) per each well, the enzyme activity is measured on a luminometer (Lumistar Galaxy, BMG Labtechnologies, Offenburg; settings: microplate: Dynatech 96, number of intervals: 50, measurement interval time: 0.2 seconds, positioning delay: 0.5 seconds, total measurement time/well: 10 seconds, gain: 250, start interval: 1, stop interval: 50).

### Cytotoxicity test

### Principle of the assay

The cytotoxicity assay analyses substances in view of toxic effects to the cells.

HeLa-P4 cells were used for the cytotoxicity assay. A commercial avaiable kit was used (ViaLight® Plus Kit, Lonza, Rockland, USA). The principle of the kit is based on measurements of the amount of ATP in the cytoplasma of metabolic active, i. e. living, cells. Any disturbance in the viability of cells results in reduction of the amount of ATP. The amount of ATP is determined by bioluminometric measurements, wherein light is formed by the enzyme luciferase from ATP and the substrate luciferin in the presence of oxygen. The intensity of emmitted light is proportional to the ATP concentration and is determined on a luminometer (Lumistar Galaxy, BMG Labtechnologies, Offenburg).

### Experimental

The test substances are prepared as described above in view of the in vitro testing of antiviral activity in the HeLa-P4 assay. Each of the substance concentrations was tested in quadruplicate.

Day 1: Plating of 1.5 x 10⁴ HeLa-P4 cells in 100 µl of medium (DMEM, 10% FCS, 2% L-glutamine, 1% penicillin/streptomycin, 500 µg/ml geniticin, 1 µg/ml puromycin) per each well of a 96-well microtiter plate, incubation by 37° C overnight.

Day 2: The medium is removed and the cells are washed with 100 µl of PBS. 78 µl of each of the substance dilutions (78, 15.6, 7.8, 3.9, 0.8, 0.4 µg/ml) and 22 µl of the medium were placed in each well of a 96-well microtiter plate and incubated for 2 h at 37 °C, the T20 dilutions (39, 19.5, 3.9, 1.95, 0.78, 0.39, 0.039 nM) are treated in the same manner and the cells are incubated for 2 days at 37 °C.

Day 4: The cells were removed from the incubator and left for 10 min at room temperature. The supernatant is removed, the cells are washed three times with 150 µl of medium per each well and lysed in 50 µl of lysis buffer (2.5 ml glycerol; 1.25 ml MES-Tris, 25 µl 1 M DTT, 250 µl Triton X100, H₂O ad 25 ml). The plate was left for 10 minutes at room temperature.

In a white micro titer plate, 100 µl of AMR-Plus reagent were placed in each well and 40 µl of the cell lysate is added (without air bubbles). The micro titer plate is incubated for 2 min at room temperature and then measured on a luminometer (Lumistar Galaxy, BMG Labtechnologies, Offenburg; settings: microplate: Dynatech 96, number of intervals: 40, measurement interval time: 0.25 seconds, positioning delay: 0.5 seconds, total measurement time/well: 10 seconds, gain: 190, start interval: 1, stop interval: 40, test type: well mode, reading direction: horizontal).

In the result, it could be seen that the Ribes extract was able to inhibit the infection caused by HIV. In the concentration used, the extract had no detectable damaging influences on the cells whatsoever. This proves an inhibitory effect of the Ribes extracts on the infectivity of HIV.

## Claims

1. Composition for the use in the prophylaxis and/or treatment of viral infections, wherein the composition comprises an aqueous extract from at least one of the aerial parts of plants of the genus Ribes, wherein the aerial parts are selected from the group consisting of leaves and twigs.

2. Composition for use according to claim 1, wherein the viral infection comprises a common cold disease.

3. Composition for use according to claim 2, wherein the common cold disease comprises a primary infection, caused by rhinoviruses, adenoviruses and/or coronaviruses.

4. Composition for use according to claim 2 or 3 for the treatment of head colds.

5. Composition for use according to claim 1, wherein the viral infection comprises influenza.

6. Composition for use according to claim 5, wherein the influenza is avian flu.

7. Composition for use according to claim 1, wherein the viral infection comprises a viral infection caused by retroviruses.

8. Composition for use according to claim 7, wherein the viral infection is caused by HIV-1 or HIV-2.

9. Composition for use according to any of the claims 1 to 8, wherein the plant is *Ribes nigrum L..*

10. Composition for use according to any of the claims 1 to 8, wherein the plant is *Ribes rubrum L..*

11. Composition for use according to any of the claims 1 to 10, wherein the composition further comprises an extract of the fruits of the plants of the genus Ribes.

12. Composition for use according to any of the claims 1 to 11, wherein the composition is in liquid, dry or semi-solid form.

13. Composition for use according to any of the claims 1 to 12, wherein the composition is administered orally, intranasally or topically.

14. Composition for use according to any of the claims 1 to 13, wherein the composition is present as a nasal agent, inhalation mixture, aerosol or room spray.

15. Composition for use according to any of the claims 1 to 14, wherein the composition is in the form of a tablet, coated tablet, effervescent tablet, capsule, powder, granulate, sugar-coated tablet, ointment, gel, cream, gargling solution or plant juice.

16. Use of an aqueous extract from at least one of the aerial parts of plants of the genus Ribes, wherein the aerial parts are selected from the group consisting of leaves and twigs, for the preparation of a medicament for the prophylaxis and/or treatment of viral infections.

17. Composition for use according to any of the claims 1 to 15, further comprising constituents of plants or plant extracts of the genus Cistus.

## Patentansprüche

1. Zusammensetzung zur Verwendung in der Prophylaxe und/oder Behandlung von Virusinfektionen, worin die Zusammensetzung einen wässrigen Extrakt von mindestens einem der oberirdischen Teile von Pflanzen der Gattung Ribes umfasst, worin die oberirdischen Teile ausgewählt sind aus der Gruppe bestehend aus Blättern und Zweigen.

2. Zusammensetzung zur Verwendung gemäß Anspruch 1, worin die Virusinfektion eine Erkältungserkrankung umfasst.

3. Zusammensetzung zur Verwendung gemäß Anspruch 2, worin die Erkältungserkrankung eine Primärinfektion, verursacht durch Rhinoviren, Adenoviren und/oder Coronaviren, umfasst.

4. Zusammensetzung zur Verwendung gemäß Anspruch 2 oder 3 für die Behandlung von Schnupfen.

5. Zusammensetzung zur Verwendung gemäß Anspruch 1, worin die Virusinfektion Grippe umfasst.

6. Zusammensetzung zur Verwendung gemäß Anspruch 5, worin die Grippe Vogelgrippe ist.

7. Zusammensetzung zur Verwendung gemäß Anspruch 1, worin die Virusinfektion eine durch Retroviren verursachte Virusinfektion umfasst.

8. Zusammensetzung zur Verwendung gemäß Anspruch 7, worin die Virusinfektion durch HIV-1 oder HIV-2 verursacht wird.

9. Zusammensetzung zur Verwendung gemäß einem der Ansprüche 1 bis 8, worin die Pflanze *Ribes nigrum* L. ist.

10. Zusammensetzung zur Verwendung gemäß einem der Ansprüche 1 bis 8, worin die Pflan*ze Ribes rubrum* L. ist.

11. Zusammensetzung zur Verwendung gemäß einem der Ansprüche 1 bis 10, worin die Zusammensetzung weiterhin einen Extrakt aus den Früchten der Pflanzen der Gattung Ribes umfasst.

12. Zusammensetzung zur Verwendung gemäß einem der Ansprüche 1 bis 11, worin die Zusammensetzung in flüssiger, trockener oder halbfester Form ist.

13. Zusammensetzung zur Verwendung gemäß einem der Ansprüche 1 bis 12, worin die Zusammensetzung oral, intranasal oder lokal verabreicht wird.

14. Zusammensetzung zur Verwendung gemäß einem der Ansprüche 1 bis 13, worin die Zusammensetzung als Nasenmittel, Inhalationsmischung, Aerosol oder Raumspray vorliegt.

15. Zusammensetzung zur Verwendung gemäß einem der Ansprüche 1 bis 14, worin die Zusammensetzung in der Form einer Tablette, beschichteten Tablette, Brausetablette, Kapsel, Pulver, Granulat, Dragee, Salbe, Gel, Creme, Gurgellösung oder Pflanzensaft vorliegt.

16. Verwendung eines wässrigen Extraktes aus mindestens einem der oberirdischen Teile der Pflanzen der Gattung Ribes, worin die oberirdischen Teile ausgewählt sind aus der Gruppe bestehend aus Blättern und Zweigen, für die Herstellung eines Arzneimittels für die Prophylaxe und/oder Behandlung von Virusinfektionen.

17. Zusammensetzung zur Verwendung gemäß einem der Ansprüche 1 bis 15, weiterhin umfassend Bestandteile von Pflanzen oder Pflanzenextrakte der Gattung Cistus.

## Revendications

1. Composition pour une utilisation dans le domaine de la prophylaxie et/ou du traitement d'infections virales, où la composition comprend un extrait aqueux d'au moins une des parties aériennes de plantes du genre Ribes, lesdites parties aériennes étant sélectionnées parmi le groupe constitué des feuilles et des rameaux.

2. Composition pour une utilisation selon la revendication 1, où l'infection virale comprend un rhume.

3. Composition pour une utilisation selon la revendication 2, où le rhume comprend une infection primaire, provoquée par des rhinovirus, des adénovirus et/ou des coronavirus.

4. Composition pour une utilisation selon la revendication 2 ou 3 pour le traitement des rhumes de cerveau.

5. Composition pour une utilisation selon la revendication 1, où l'infection virale comprend la grippe de type A.

6. Composition pour une utilisation selon la revendication 5, où la grippe de type A est une grippe aviaire.

7. Composition pour une utilisation selon la revendication 1, où l'infection virale comprend une infection virale provoquée par des rétrovirus.

8. Composition pour une utilisation selon la revendication 7, où l'infection virale est provoquée par le VIH-1 ou le VIH-2.

9. Composition pour une utilisation selon l'une quelconque des revendications 1 à 8, où la plante est *Ribes nigrum L.*

10. Composition pour une utilisation selon l'une quelconque des revendications 1 à 8, où la plante est *Ribes rubrum L.*

11. Composition pour une utilisation selon l'une quelconque des revendications 1 à 10, où la composition comprend en outre un extrait des fruits des plantes du genre Ribes.

12. Composition pour une utilisation selon l'une quelconque des revendications 1 à 11, où la composition est sous forme liquide, de poudre ou semi-solide.

13. Composition pour une utilisation selon l'une quelconque des revendications 1 à 12, où la composition est administrée par voie orale, intranasale ou topique.

14. Composition pour une utilisation selon l'une quelconque des revendications 1 à 13, où la composition se présente sous la forme d'un produit actif pour le nez, d'un mélange à inhaler, d'un aérosol ou d'un vaporisateur d'atmosphère.

15. Composition pour une utilisation selon l'une quelconque des revendications 1 à 4, où la composition est sous la forme d'un comprimé, d'un comprimé enrobé, d'un comprimé effervescent, d'une gélule, d'une poudre, d'un granule, d'un comprimé dragéifié, d'un onguent, d'un gel, d'une crème, d'une solution pour bain de bouche ou d'un jus de plantes.

16. Utilisation d'un extrait aqueux d'au moins une des parties aériennes de plantes du genre Ribes, où les parties aériennes sont sélectionnées parmi le groupe constitué des feuilles et des rameaux, pour la préparation d'un médicament destiné à la prophylaxie et/ou au traitement d'infections virales.

17. Composition pour une utilisation selon l'une quelconque des revendications 1 à 15, comprenant en outre des constituants de plantes ou des extraits de plantes du genre Cistus.
